# EUROPEAN PATENT APPLICATION

(11) **EP 4 389 140 A1**
(43) Date of publication of application: **26.06.2024**
(21) Application number: 23211679.8
(22) Date of filing: 23.11.2023
(51) Int. Cl.: A61K 38/48, A61K 47/16, C12Q 1/56, G01N 33/86

(54) **LIQUID THROMBIN REAGENT CONTAINING GUANIDINE DERIVATIVES**

(30) Priority: 23.12.2022 US 202218088068
(71) Applicant: Instrumentation Laboratory Company, Bedford, MA 01730 (US)
(72) Inventor: CAO, Zhenghua, Bedford, 01730 (US); KUNG, Chun, Bedford, 01730 (US); BOTTENUS, Ralph, Bedford, 01730 (US)
(74) Representative: Müller-Boré & Partner Patentanwälte PartG mbB

(57) **Abstract**

An example composition that is liquid includes thrombin and a constituent that includes one or more of guanidine derivatives or one or more anticoagulants. The one or more guanidine derivative may include 1-methylguanidine, 1,1-dimethylguanidine, 1,1-diethylguanidine, or N-benzyl-N-methylguanidine. The one or more anticoagulants may include rivaroxaban, apixaban, edoxaban, betrixaban, or a factor Xa anticoagulant.

## Description

### TECHNICAL FIELD

This specification relates generally to example liquid thrombin reagent compositions.

### BACKGROUND

In a test sample, such as whole blood, thrombin triggers the formation of densely-packed fibrin clots comprised of thin fibrin fibers. Thrombin may be incorporated into a reagent to initiate clot formation in the test sample.

Thrombin reagent may be in lyophilized form or liquid form. For thrombin reagents in liquid form, the stability of the reagent can be adversely affected by the inherent autolysis and denaturation properties of thrombin in the liquid.

### SUMMARY

An example composition includes thrombin and a constituent that includes one or more of guanidine derivatives or one or more anticoagulants. The example composition is liquid. The example composition may include one or more of the following features, either alone or in combination.

The one or more guanidine derivatives may include a mono-alkylated guanidine derivative. The one or more guanidine derivatives may include a dialkylated guanidine derivative. The one or more guanidine derivatives may include 1-methylguanidine, 1,1-dimethylguanidine, 1,1-diethylguanidine, or N-benzyl-N-methylguanidine, where N indicates a substituent on nitrogen. The one or more guanidine derivatives may include alkylated guanidine having a chemical structure represented by formula: 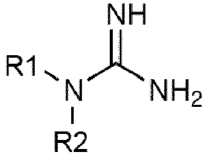 where R1 is H, methyl, ethyl, or benzyl, where R2 is H, methyl, ethyl, or benzyl, and where N is nitrogen and H is hydrogen.

The one or more guanidine derivatives may include a mono-alkylated guanidine including an alkyl group having a length that is different from a length of an alkyl group of 1-methylguanidine, or a dialkylated guanidine including an alkyl group having a length that is different from a length of an alkyl group of 1,1 -dimethylguanidine or 1,1-diethylguanidine. The one or more guanidine derivatives may include a sulfate salt.

The one or more guanidine derivatives may have a concentration in the composition of about 0.5 millimolar (mM) or greater for every unit per milliliter (U/mL) of thrombin. The one or more guanidine derivatives may have a concentration in the composition of about 1 mM or greater for every unit per milliliter of thrombin. The one or more guanidine derivatives may have a concentration in the composition of about 20 mM (millimolar) or greater. The thrombin may have a concentration in the composition of about 2 U/mL (units-per-milliliter) or greater or a concentration of about 20 U/mL (units-per-milliliter) or greater. The one or more guanidine derivatives may have a concentration in the composition of about 70 mM (millimolar) or greater.

The one or more anticoagulants may include rivaroxaban, apixaban, edoxaban, betrixaban, or a factor Xa anti-coagulant.

The composition may include a buffer that is aqueous. The buffer may include 2-morpholin-4-ylethanesulfonic acid (MES) and a preservative. The buffer may include one or more carboxylic acid derivatives.

The composition may be a liquid reagent for use as (i) a Clauss reagent, (ii) a thrombin time reagent, or (iii) a diluted thrombin reagent for direct thrombin inhibitors.

The composition may a water-based solution. The composition may be liquid at least at 20°C. The composition may be liquid at least at 37°C.

An example method includes the following operations: forming a mixture including a liquid reagent and a sample, where the sample includes whole blood, part of the whole blood, or a derivative of the whole blood, and where the liquid reagent includes: thrombin and a guanidine derivative. The method may include determining an amount of fibrinogen or an amount of anticoagulant directed against thrombin in the mixture. The method may include one or more of the following features, either alone or in combination.

The liquid reagent and the liquid mixture may be in a cartridge. The fibrinogen or anticoagulant directed against thrombin may be measured by an instrument in which the cartridge is inserted. The liquid reagent may include a chromogenic substrate susceptible to cleavage by thrombin.

An example method of measuring a thrombin inhibitor in a sample may include the following operations: forming a mixture that includes a liquid reagent and the sample, where the sample includes the thrombin inhibitor in whole blood, part of the whole blood, or a derivative of the whole blood, and where the liquid reagent includes: thrombin and a guanidine derivative. The method also includes determining an amount of the thrombin inhibitor in the mixture. The guanidine derivative may include 1-methylguanidine, 1,1-dimethylguanidine, 1,1-diethylguanidine, or N-benzyl-N-methylguanidine. Examples of thrombin inhibitors that may be determined include DOACs (direct oral anticoagulant) directed against thrombin, such as dabigatran. Other examples of thrombin inhibitors include direct thrombin inhibitors (DTIs) such as lepirudin, desirudin, bivalirudin, and argatroban.

Any two or more of the features described in this specification, including in this summary section, can be combined to form implementations not specifically described herein.

The systems and processes described herein, or portions thereof, may be implemented as one or more apparatus or as a method and may include one or more processing devices and computer memory to store executable instructions to implement control of various functions. The systems, processes, and substances, including but not limited to apparatus, methods, and/or reagents, described herein may be configured, for example, through design, manufacture, construction, composition, arrangement, placement, programming, operation, activation, deactivation, and/or control.

The details of one or more implementations are set forth in the accompanying drawings and the description below. Other features, objects, and advantages will be apparent from the description and drawings, and from the claims.

### DESCRIPTION OF THE DRAWINGS

Figs. 1A to 1F show structural formulas of examples of guanidine derivatives that may be used in the example liquid thrombin reagents described herein.
Fig. 2A shows a plot of α-thrombin activity and α/β-thrombin activity over time obtained using a clotting assay.
Fig. 2B shows a plot showing α-thrombin activity and α/β-thrombin activity over time obtained using a chromogenic assay.
Fig. 3 is a plot showing time-to-clot (TTC) for a test sample mixed with a liquid thrombin reagent containing apixaban and for a test sample mixed with a liquid thrombin reagent not containing apixaban.
Fig. 4 is a plot showing factor Xa inhibition for different concentrations of guanidine and guanidine derivatives.
Fig. 5 is a plot showing thrombin activity over time for a test sample containing guanidine derivatives and for a test sample not containing a guanidine derivative.
Fig. 6 is a plot showing remaining thrombin activity for liquid thrombin reagents containing different concentrations of dimethylguanidine after storage at 37°C.
Fig. 7 is a plot showing remaining thrombin activity for liquid thrombin reagents containing different thrombin concentrations in the presence of a guanidine derivative after storage at 37°C.
Fig. 8A is a flowchart showing an example process for measuring a concentration of fibrinogen in a test sample using a clotting assay.
Fig. 8B is a flowchart showing an example process for measuring a concentration of thrombin inhibitor in a test sample using a clotting assay.
Fig. 8C is a flowchart showing an example process for measuring a concentration of an anticoagulant directed against thrombin in a test sample using a fluorogenic assay.
Fig. 8D is a flowchart showing an example process for measuring a concentration of an anticoagulant directed against thrombin in a test sample using a chromogenic assay.

Like reference numerals in different figures indicate like elements

### DETAILED DESCRIPTION

Described herein are examples of thrombin reagents that may be liquid over a temperature range of at least 0° Celsius (C) to 100° C, and that are stable over at least a typical reagent storage temperature range, usage temperature range, and/or room temperature. All temperatures described herein are at a standard pressure of 1 atmosphere (101.325 kilopascal). "About" as used herein allows for a deviation from a given number, such as, but not limited to, a deviation of 1%, 2%, 3%, 4%, or 5%.

A liquid thrombin reagent may be characterized as stable if liquid thrombin therein retains about 70% to about 100% of initial thrombin activity over a time period. Initial thrombin activity in this context includes activity of the liquid thrombin before the liquid thrombin reagent is subjected to one or more conditions that cause, enable, or promote thrombin autolysis. Examples of such activities include, but are not limited to, storage of the liquid thrombin reagent for more than one day at room temperature of about 20°C, storage of the liquid thrombin reagent for more than one month in a temperature range of about 2°C to about 8°C or about 2°C to about 45°C, and/or testing using the liquid thrombin reagent at body temperature of about 37°C.

An example liquid thrombin reagent includes thrombin, an aqueous buffer, and one or more constituents that improve the stability of thrombin relative to a liquid thrombin reagent that does not include those one or more constituents. The stability of a liquid thrombin reagent may be achieved through reduction in autolysis of thrombin in the above example temperature ranges without significant reductions in the liquid thrombin reagent's biological activity.

In some examples, the concentration of thrombin in the liquid thrombin reagent may be within a range of about 2 units-per-milliliter (U/mL) to about 20 U/mL inclusive. In some examples, the concentration of thrombin in the liquid thrombin reagent may be within a range of about 7 U/mL to about 70 U/mL inclusive. In a particular, non-limiting examples, the concentration of thrombin in the liquid thrombin reagent may be about 7 U/ml, or about 15 U/mL, or about 20 U/ml, or about 70 U/ml. The concentration of thrombin in the liquid thrombin reagent is not limited to the values or ranges presented above. For instance, in another non-limiting example, the concentration of thrombin in the liquid thrombin is about 100 U/ml.

Examples of the one or more constituents that may be contained in the liquid thrombin reagent to improve the stability of thrombin, and thus of the liquid thrombin reagent itself, include one or more guanidine derivatives, one or more anticoagulants, or a combination of the one or more guanidine derivatives and the one or more anticoagulants.

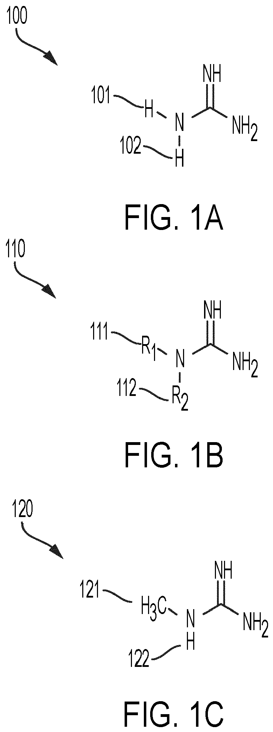

Guanidine 100 is a nitrogen-rich organic compound having the formula HNC(NH₂)₂. The structural formula of guanidine 100 is shown in Fig. 1A.

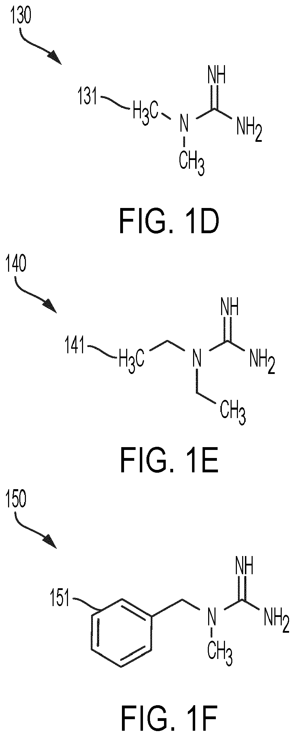

An example guanidine derivative that may be contained in the liquid thrombin reagent to improve the stability of liquid thrombin may be an alkylated guanidine. An alkylated guanidine may have one or both of the hydrogen (H) atoms 101 and 102 in guanidine 100 replaced with a larger chemical group, represented as R1 111 and R2 112 in Fig. 1B, where R1 111 may be a methyl group 131 (Fig. 1D), an ethyl group 141 (Fig. 1E), or a benzyl group 151 (Fig. 1F); and R2 112 may be a methyl group 132, an ethyl group 142, or a benzyl group 151. A methyl group 131 includes an alkyl derived from methane, containing one carbon atom bonded to three hydrogen atoms, having the chemical formula -CH₃. An ethyl group 141 includes an alkyl substituent with the formula -CH₂CH₃, derived from ethane (C₂H₆). A benzyl group 151 includes a substituent or molecular fragment having the structure R-CH₂-C₆H₅. Benzyl features a phenyl group(C₆H₅) attached to a methylene group (-CH₂-) group.

Other examples of guanidine derivatives that may be included in the liquid thrombin reagent to improve the stability of thrombin may include a mono-alkylated guanidine, a dialkylated guanidine, or a combination of a mono-alkylated guanidine and a dialkylated guanidine. Non-limiting examples of guanidine derivatives that may be included in the liquid thrombin reagent may include: 1-methylguanidine, 1,1-dimethylguanidine, 1,1-diethylguanidine, or N-benzyl-N-methylguanidine, where N indicates a substituent on nitrogen. Fig. 1C shows the structural formula for 1-methylguanidine 120, Fig. 1D shows the structural formula for 1,1-dimethylguanidine 130, Fig. 1E shows the structural formula for 1,1-diethylguanidine 140, and Fig. 1F shows the structural formula for N-benzyl-N-methylguanidine 150.

Other examples of guanidine derivatives that may be included in the liquid thrombin reagent to improve the stability of thrombin may include a mono-alkylated guanidine derivative that contains an alkyl group that is different in length than the alkyl group in 1-methylguanidine 120 of Fig. 1C. For example, an alkyl group that is different in length than the alkyl group in 1-methylguanidine 120 may include two or more carbon atoms. An alkyl group with two carbon atoms has the formula -CH₂CH₃. A mono-alkylated guanidine derivative that contains an alkyl group with two carbon atoms has the formula C₃H₉N₃. An alkyl group with three carbon atoms has the formula -CH₂CH₂CH₃. A mono-alkylated guanidine derivative that contains an alkyl group with three carbon atoms has the formula C₄H₁₁N₃. An alkyl group with four carbon atoms has the formula -CH₂CH₂CH₂CH₃. A mono-alkylated guanidine derivative that contains an alkyl group with four carbon atoms has the formula C₅H₁₃N₃.

Other examples of guanidine derivatives that may be included in the liquid thrombin reagent to improve the stability of thrombin may include dialkylated guanidine derivatives that contains one or both alkyl groups that are different in length than the alkyl groups in 1,1-dimethylguanidine 130 of Fig. 1D. Alkyl groups that are different in length than the alkyl groups in 1,1-dimethylguanidine 130 may include one or more carbon atoms. An alkyl group with one carbon has the formula -CH₃. A dialkylated guanidine derivative that contains one alkyl group with one carbon and one alkyl group with two carbon atoms has the formula C₄H₁₁N₃.

Other examples of guanidine derivatives that may be included in the liquid thrombin reagent to improve the stability of thrombin may include dialkylated guanidine derivatives that contains one or both alkyl groups that are different in length than 1,1-diethylguanidine 140 of Fig. 1E. Alkyl groups that are different in length than the alkyl groups in 1,1-diethylguanidine 140 may include three or more carbon atoms. An alkyl group with three carbon atoms has the formula -CH₂CH₂CH₃. A dialkylated guanidine derivative that contains a one alkyl group with two carbon atoms and one alkyl group with three carbon atoms has the formula C₆H₁₅N₃. An alkyl group with four carbon atoms has the formula -CH₂CH₂CH₂CH₃. A dialkylated guanidine derivative that contains one alkyl group with two carbon and one alkyl group with four carbon atoms has the formula C₇H₁₇N₃.

Other examples of guanidine derivatives that may be included in the liquid thrombin reagent to improve the stability of thrombin may include a dialkylated guanidine derivative that contains one alkyl group that is longer in length than the alkyl groups in 1,1-dimethylguanidine 130 or 1,1-diethylguanidine 140 and has another alkyl group that is the same length or shorter than the alkyl groups in 1,1-dimethylguanidine 130 or 1,1-diethylguanidine 140. An alkyl group with the same length as the alkyl groups in 1,1-dimethylguaninde 130 has the chemical formula -CH₃. An alkyl group with the same length as the alkyl groups in 1,1-diethylguaninde 140 has the chemical formula -CH₂CH₃.

Other examples of guanidine derivatives that may be included in the liquid thrombin reagent to improve the stability of thrombin may include a salt containing a conjugate acid of guanidine, which has the chemical formula C(NH₂)₃⁺. Examples of guanidine-derivative salts that may be included in the liquid thrombin reagent may include a sulfate salt containing a conjugate acid of guanidine, a carbonate salt containing a conjugate acid of guanidine, a chloride salt containing a conjugate acid of guanidine, a nitrate salt containing a conjugate acid of guanidine, a perchlorate salt containing a conjugate acid of guanidine, and/or a picrate salt containing a conjugate acid of guanidine.

The guanidine derivatives that may be included in the liquid thrombin reagent to improve the stability of thrombin may include a combination of two or more of the example guanidine derivatives described herein. Example combinations of guanidine derivates that may be included in the liquid thrombin regent may be a combination of 1-methylguanidine 120 and 1,1-dimethylguanidine 130, a combination of 1-methylguanidine 120 and 1,1-diethylguanidine 140, a combination of 1-methylguanidine 120 and N-benzyl-N-methylguanidine 150, a combination of 1,1-dimethylguanidine 130 and 1,1-diethylguanidine 140, a combination of 1,1-diethylguanidine 140 and N-benzyl-N-methylguanidine 150, and/or a combination of 1,1-dimethylguanidine 130 and N-benzyl-N-methylguanidine 150.

The guanidine derivatives that may be included in the liquid thrombin reagent to improve the stability of thrombin may include a combination of one or more of the example guanidine derivatives described herein and guanidine itself (Fig. 1A). Example combinations of guanidine and guanidine derivates that may be included in the liquid thrombin regent may be a combination of guanidine and 1-methylguanidine, a combination of guanidine and 1,1-dimethylguanidine, a combination of guanidine and 1,1-diethylguanidine, and/or a combination of guanidine and N-benzyl-N-methylguanidine.

In some implementations, individual guanidine derivatives described herein may each be present in the liquid thrombin reagent at a concentration of about 0.5 millimolar (mM) or greater for every unit per milliliter (U/mL) of thrombin. In some implementations, individual guanidine derivatives described herein may each be present in the liquid thrombin reagent at a concentration of about 1 mM or greater for every unit per milliliter of thrombin. In some implementations, individual guanidine derivatives described herein may each be present in the liquid thrombin reagent at a concentration of about 2 mM to about 20 mM inclusive or at a concentration of 20 mM or greater. In some implementations, a combination of two or more of the guanidine derivatives described herein may be present in the liquid thrombin reagent at a concentration of about 2 mM to about 20 mM inclusive or at a concentration of 20 mM or greater.

The one or more constituents that may be added to the liquid thrombin reagent to improve the stability of thrombin may be or include one or more anticoagulants. Examples of anticoagulants that may be added to the liquid thrombin reagent include known factor Xa inhibitors such as rivaroxaban, apixaban, edoxaban, and betrixaban. These anticoagulants may be added to the liquid thrombin reagent individually or in a combination of two or more of them. Example combinations of anticoagulants that may be included in the liquid thrombin regent may include, but are not limited to, a combination of apixaban and rivaroxaban, a combination of apixaban and edoxaban, a combination of apixaban and betrixaban, a combination of rivaroxaban and betrixaban, a combination of edoxaban and betrixaban, and/or a combination of edoxaban and rivaroxaban.

In some implementations, individual anticoagulants described herein may each be present in the liquid thrombin reagent at a concentration of about 20 nanograms per milliliter (ng/mL) to about 1000 ng/mL of anticoagulant, inclusive.

In some implementations, the liquid thrombin reagent may include one or more of the guanidine derivatives described herein and one or more of the anticoagulants described herein. Example combinations of guanidine derivatives and factor Xa anticoagulants that may be included in the liquid thrombin reagent include, but are not limited to, a combination of 1-methylguanidine 120 and apixaban, a combination of 1,1-dimethylguanidine 130 and apixaban, a combination of 1,1-diethylguanidine 140 and apixaban, a combination of 1,1-dimethylguanidine 130 and rivaroxaban, a combination of 1,1-dimethylguanidine 130 and edoxaban, and/or a combination of 1,1-dimethylguanidine 130 and betrixaban.

In some implementations the aqueous buffer in the liquid thrombin reagent may be or include 2-morpholin-4-ylethanesulfonic acid (MES) or 3-(N-morpholino) propanesulfonic acid (MOPS), which is a structural analog to MES. In some implementations the aqueous buffer in the liquid thrombin reagent may be or include HEPES ((4-(2-hydroxyethyl)-1-piperazineethanesulfonic acid), Bis-Tris buffer, citrate, ADA (N-(2-acetamido)iminodiacetic acid, N-(carbamoylmethyl)iminodiacetic acid) buffer, ACES (N-(2-Acetamido)-2-aminoethanesulfonic acid) buffer, PIPES (piperazine-N,N'-bis(2-ethanesulfonic acid)) buffer, imidazole/imidazolium buffer, Bis-Tris Propane buffer, maleic acid buffer, phosphate buffer, MOPSO (2-Hydroxy-3-morpholinopropanesulfonic acid) buffer, BES (bis(2-hydroxyethyl)-2-amino-ethansulfonic acid) buffer, MOPS buffer, TES (tris(hydroxymethyl)methyl-2-aminomethane sulfonic acid) buffer, and/or MOBS (3-(N-morpholino)propanesulfonic acid) buffer.

In some implementations the aqueous buffer in the liquid thrombin reagent may be or include one or more carboxylic acid derivatives. Examples of carboxylic acid derivatives that may be included in the aqueous buffer may include the carboxylic acid salt acetates, ethylene diamine tetra acetate, butane tetra carboxylate, propane tricarboxylate, citrate, succinate, tartarate, malonate, and/or gluconate.

In some implementations the aqueous buffer in the liquid thrombin reagent may include a preservative. Examples of preservatives that may be included in the aqueous buffer may include ProCiin^{™} 300 (3% 5-chloro-2-methyl-4-isothiazolin-3-one (CMIT) and 2-methyl-4-isothiazloin-3-one (MIT)), sodium azide, gentamicin, thimerosal, butylated hydroxytoluene (BHT), sucrose, trehalose, glycerin, sodium citrate, poloxamer, cetyl trimethyl ammonium bromide (CTAB), or a combination of two or more of these.

In some implementations one or more substrates may be added to a mixture of the liquid thrombin reagent and a test sample to measure the amount of thrombin inhibitor in a test sample. Examples of substrates that may be added to the liquid thrombin reagent include the chromogenic substrates H-D-phenylalanyl-L-pipecolyl-L-arginine- p-nitroaniline dihydrochloride (H-D-Phe-Pip-Arg-pNA) (Chromogenix S-2238^{™}) and L-pyroglutamyl-L-prolyl-L-argininep-nitroaniline hydrochloride (Chromogenix S-2366^{™}), or the fluorogenic substrates Z-Gly-Gly-Arg-AMC, Technothrombin^{®}, and/or Calibrated Automated Thrombogram.

### CLOTTING ASSAYS

A clotting-based assay can be used to determine a concentration of fibrinogen (with an excess amount of thrombin) in a test sample and/or to determine a concentration of thrombin inhibitor (with thrombin acting as a rate-limiting material) in a test sample. In this regard, fibrinogen is a soluble protein that circulates in the blood and, upon vascular injury, is enzymatically converted to fibrin by thrombin to begin the clotting process. Examples of thrombin inhibitors that may be determined include DOACs (direct oral anticoagulant) directed against thrombin, such as dabigatran. Other examples of thrombin inhibitors include direct thrombin inhibitors (DTIs) such as lepirudin, desirudin, bivalirudin, and argatroban.

The test sample may be whole blood, a part of whole blood, a sample derived from whole blood, a part of whole blood that has been depleted of one or more blood constituents (e.g., depleted of leukocytes, erythrocytes, platelets, or protein (e.g., albumin)), or a constituent of whole blood that has been isolated. In an example, test sample may be platelet-free plasma, which is whole blood part that has been depleted of a blood constituent (platelets). The test sample may be collected from a subject, such as a human, using standard techniques.

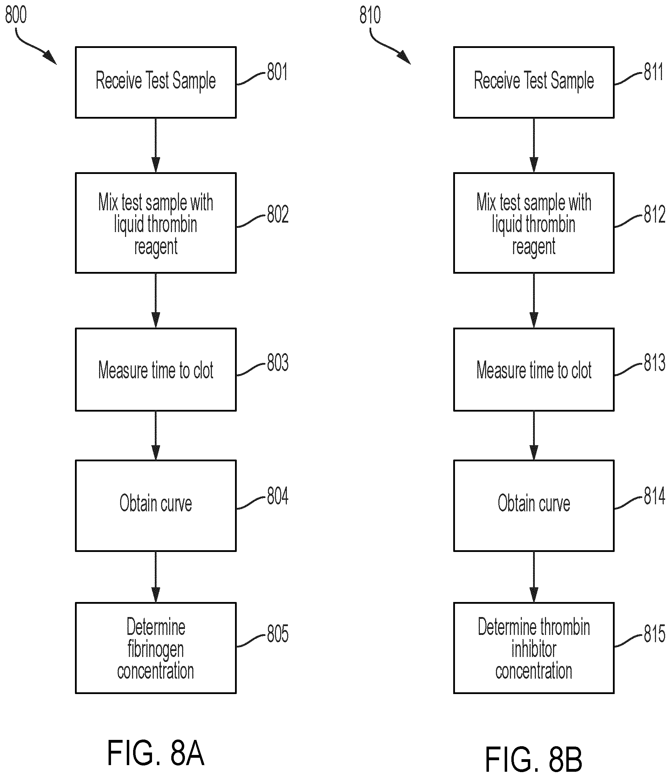

Fig. 8A illustrates an example process 800 that may be performed to determine the fibrinogen concentration associated with a test sample using a clotting assay. The clotting assay determines the time for a test sample mixed with the liquid thrombin reagent to clot. According to process 800, the test sample is received (801) and is mixed (802) with a liquid thrombin reagent of the type described herein. The TTC (time-to-clot) of the resulting mixture is measured (803). In this example, the TTC of a series of samples having known fibrinogen concentrations were previously measured and used to create a standard curve that relates TTC values to fibrinogen concentrations, which is obtained (e.g., accesses from memory) (804). The fibrinogen concentration in the blood sample is determined (805) by comparing the TTC of the mixture with the standard curve of the known fibrinogen concentrations samples. That is, the TTC of the mixture is identified on the standard curve and the corresponding fibrinogen concentration is obtained from the standard curve. However, determining fibrinogen concentration is not limited to processes using a standard curve. In some implementations, additional methods, for example machine learning algorithms, use the results of a clotting assay (e.g., TTC and/or other parameters derived from the results) to derive the fibrinogen concentration in the test sample.

Fig. 8B illustrates an example process 810 that may be performed to determine a thrombin inhibitor concentration associated with a test sample using a clotting assay. The clotting assay determines the time for a test sample mixed with the liquid thrombin reagent to clot. According to process 800, the test sample is received (811) and is mixed (812) with a liquid thrombin reagent of the type described herein. The TTC of the resulting mixture is measured (813). In this example, the TTC of a series of samples having known thrombin inhibitor concentrations were previously measured and used to create a standard curve that relates TTC values to thrombin inhibitor concentrations, which is obtained in operation 814. The thrombin inhibitor in the blood sample is determined (815) by comparing the TTC of the mixture with the standard curve of the known thrombin inhibitor concentrations samples. That is, the TTC of the mixture is identified on the standard curve and the corresponding thrombin inhibitor concentration is obtained from the standard curve. However, determining thrombin inhibitor concentration is not limited to processes using a standard curve. In some implementations, additional methods, for example machine learning algorithms, use the results of a clotting assay (e.g., TTC and/or other parameters derived from the results) to derive the thrombin inhibitor concentration in the test sample.

An example cartridge used in a blood testing system may be used to perform the clotting assays and may include one or more chambers for receiving/containing the test sample, the reagent(s), and/or the mixture of the test sample and the reagent(s). A clotting assay is performed on the mixture of the test sample and reagent to determine clotting characteristic(s) based on the mixture, such as TTC. The cartridge may include multiple fluid channels to allow for and facilitate fluidic movement of the sample or other materials in the cartridge, e.g., controlled by pressure or other mechanisms.

In a non-limiting example, the clotting assays may be performed using the system described in in U.S. Patent No. 10,175,225 ("Blood Testing System and Method") that issued on January 8, 2019, the contents of which are incorporated herein by reference. U.S. Patent No. 10,175,225 describes a blood testing system that include an analyzer console and one or more cartridges for performing tests, including a test to determine the amount of fibrinogen in a test sample using a liquid thrombin reagent such as those described herein. The testing may be performed using a cup and pin structure as used in Rotem^{®} *sigma* and Rotem^{®} *delta* systems provided by Werfen^{®} S.A. The clotting characteristic(s) may be used by the associated analyzer console to implement the processes described herein for determining the amount of fibrinogen in the mixture and/or determining an amount thrombin inhibitor in the mixture.

In another non-limiting example, the clotting assays may be performed using the system described in U.S. Patent Publication No. 2018/0306774 ("Disposable System for Analysis of Hemostatic Function") that published on October 25, 2018, the contents of which are incorporated herein by reference. This patent publication describes Hemosonics^{®} Quantra^{®} hemostasis analyzer.

In still another non-limiting example, the clotting assays may be performed using the TEG^{®} 6s cartridge-based system provided by Haemonetics^{®}. That system includes a cartridge containing one or more chambers that may hold a mixture of test sample such as blood and the liquid thrombin reagent described herein. Testing in the TEG^{®} 6s system is performed using optical analysis in combination with resonant frequency technology and is used to determine clotting characteristic(s) based on the mixture such as TTC. More specifically, in examples using the TEG^{®} 6s cartridge-based system, as described in Haemonetics^{®} USFDA 501K Summary, "The TEG^{®} 6s technology is based on a disposable cartridge containing up to 4 independent measurement cells. Each cell consists of a short vertically-oriented injection molded tube (ring). Detection of clotting in the TEG^{®} 6s Hemostasis System is performed optically. A piezoelectric actuator vibrates the measurement cell(s) through a motion profile composed of summed sinusoids at different frequencies. The movement of the measurement cells will induce motion in the sample meniscus, which will be detected by a photodiode. The resulting motion of the meniscus is detected optically and analyzed by the instrument to calculate the resonant frequency and modulus of elasticity (stiffness) of the sample. By performing a Fast Fourier Transform (FFT) on meniscus motion data, the resonant frequencies can be determined. The analyzer detects the harmonic motion of a hanging drop of blood in response to external vibration. As the sample transitions from a liquid state to a gel-like state during clotting, the modulus of elasticity (stiffness) and therefore resonant frequency increase. A TEG^{®} 6s hemostasis analyzer measures these variations in resonant frequency during clotting and lysis." The clotting characteristic(s) determined using the TEG^{®} 6s technology may be used by analyzer to implement the processes described herein for determining the amount of fibrinogen in the mixture and/or determining an amount of thrombin inhibitor in the mixture.

In still another non-limiting example, the clotting assays may be performed on the VerifyNow^{®} system provided by Werfen^{®} S.A.

### CHROMOGENIC AND FLUOROGENIC ASSAYS

In some implementations, a concentration of thrombin inhibitor in a test sample may be determined using a chromogenic or fluorogenic assay. In such an assay, the thrombin inhibitor inhibits activity of thrombin in a reaction mixture containing the test sample. The assay measures any remaining free thrombin in the mixture based on cleavage of the free thrombin to the chromogenic or fluorogenic substrate. The chromogenic assay uses colored substrates to quantify the activity of the free thrombin. The fluorogenic assay uses fluorescent substrates to quantify the activity of the free thrombin. In each assay, one or more curves is generated based on the amount of thrombin activity as defined by fluorescence values or absorbance values and the amount of thrombin inhibitor in a reaction mixture.

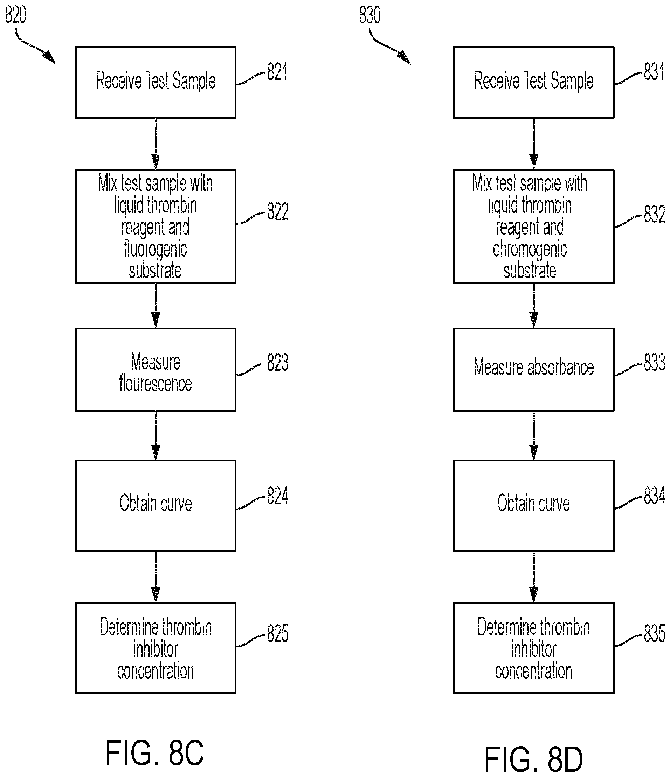

Fig. 8C illustrates an example process 820 that may be performed to determine the thrombin inhibitor concentration associated with a blood sample using the fluorogenic assay. The test sample is received (821) and is mixed (822) with a liquid thrombin reagent and a fluorogenic substrate. The fluorescence of the cleaved fluorogenic substrate in the resulting mixture is measured (823). In this example, the fluorescence of a series of samples having known thrombin inhibitor concentrations were previously measured and used to create a standard curve described above that relates fluorescence values to thrombin inhibitor concentrations, which is obtained in operation 824. The thrombin inhibitor concentration in the test sample is determined (825) by comparing the fluorescence of the mixture with the standard curve of the known thrombin inhibitor concentration samples. That is, the fluorescence of the mixture is identified on the standard curve and the corresponding thrombin inhibitor concentration is obtained from the standard curve. However, determining thrombin inhibitor concentration is not limited to processes using a standard curve prepared in this example. In some implementations, additional methods, for example machine learning algorithms, use the results of a fluorogenic assay (e.g., fluorescence and/or other parameters derived from the results) to derive the thrombin inhibitor concentration in the test sample.

In some implementations, fluorogenic assays may be performed on the PHERAstar^{®} FSX fluorescence microplate reader provided by BMG LABTECH^{®} Inc. That system includes a microplate-based system for reading high throughput, multimode reading of test samples and the liquid thrombin reagents described herein. In another non-limiting example, fluorescent assays may be performed on the Xenius XOF spectrofluorometer provided by SAFAS^{®} Ltd. That system includes a microplate- or cuvette-based system for reading *in situ,* high throughput reading of test samples and the liquid thrombin reagents described herein.

Fig. 8D illustrates an example process 830 that may be performed to determine the thrombin inhibitor concentration in a test sample using a chromogenic assay. According to process 830, a test sample is received (831) and is mixed (832) with a chromogenic substrate and a liquid thrombin reagent of the type described herein. An absorbance of the cleaved chromogenic substrate in the resulting mixture is spectrophotometrically measured (833). In this example, the absorbance of a series of samples having known thrombin inhibitor concentrations were previously measured and used to create a standard curve described above that relates absorbance values to thrombin inhibitor concentrations, which is obtained in operation 834. The thrombin inhibitor concentration in the test sample is determined (835) by comparing the absorbance of the mixture with the standard curve of the known thrombin inhibitor concentration samples. That is, the absorbance of the mixture is identified on the standard curve and the corresponding thrombin inhibitor concentration is obtained from the standard curve. However, determining the thrombin inhibitor concentration is not limited to processes using a standard curve prepared in this example. In some implementations, additional methods, for example machine learning algorithms, use the results of a chromogenic assay (e.g., absorbance and/or other parameters derived from the results) to derive the thrombin inhibitor concentration in the test sample.

In some implementations, chromogenic assays may be performed on an ACL TOP^{®} Family hemostasis testing system provided by Werfen^{®} S.A. That system includes a fully automated system for loading and unloading cuvettes hold a mixture of test sample such as blood and the liquid thrombin reagent described herein. In another non-limiting example, chromogenic assays may be performed on a SPECTROstar Nano^{®} absorbance microplate reader provided by BMG LABTECH^{®} Inc. That system includes a microplate- or cuvette-based system for reading full-spectrum absorbance of test samples and the liquid thrombin reagents described herein.

### TESTING THROMBIN ACTIVITY IN LIQUID THROMBIN REAGENT

This experiment is presented to show the stability of the liquid thrombin reagent using a clotting assay and a chromogenic assay.

In the clotting assay, the liquid thrombin reagent was diluted with an aqueous buffer to a final thrombin concentration of within 5 to 15 U/mL of thrombin. Then, 20 to 40 microliters (µL) of the diluted liquid thrombin reagent was mixed with 80 µL of a reaction buffer containing 20 mM HEPES, pH 7.4, containing 150 mM NaCl and 0.5% BSA (bovine serum albumin). After an incubation period of 60 to 90 seconds at 37°C, 80 µL of normal pooled plasma was added to the mixture and the time to clot was determined using a threshold algorithm.

In the chromogenic assay, the liquid thrombin reagent was diluted with an aqueous buffer to a final thrombin concentration of within 1-5 U/mL thrombin. Then, 20 µL of the diluted liquid thrombin reagent was mixed with 90 µL of the same reaction buffer used for clotting assay. After an incubation period of 60 to 90 seconds at 37°C, 90 µL of 0.2 mM of thrombin substrate S-2238 (H-D-Phe-Pip-Arg-pNA) prepared in the reaction buffer was added to the mixture and the reaction rate of the liquid thrombin reagent was determined using a linear kinetics algorithm.

To standardize thrombin activity, the time to clot determined by the clotting assay and the reaction rate determined by the chromogenic assay calculated thrombin activities were compared to calibration reaction rate curves from human α-thrombin or thrombin standards commercially available from the National Institute for Biological Standards and Control (NIBSC).

### EFFECT OF β THROMBIN ON LIQUID THROMBIN REAGENT STABILITY

β-thrombin and γ-thrombin are known to degrade α-thrombin in liquid thrombin reagents. Specifically, β-thrombin(s) in liquid thrombin reagents will increase the autolytic activity of α-thrombin, and lead to a loss in α-thrombin activity, e.g., once the β-thrombin content exceeds 30% of the total thrombin content.

To show that the β-thrombin(s) under certain conditions have little or no effect on the liquid thrombin reagents described herein, the following experiment was performed. The effects of β-thrombin were analyzed using clotting and chromogenic assays.

In this experiment, a liquid thrombin reagent of the type described herein containing α-thrombin was diluted with an aqueous buffer to a final concentration of 100 U/mL α-thrombin. A liquid thrombin reagent of the type described herein containing both α-thrombin and β-thrombin (α/β-thrombin) was diluted with an aqueous buffer to a final concentration of 100 U/mL α-thrombin and 50 U/mL β-thrombin. In this experiment, the aqueous buffer contained 20 mM MES, pH 6.3, 100 mM NaCl, 25 mM sodium acetate, 25 mM sodium succinate, 5 mM CaCl₂, 0.5% BSA, and 0.4 g/L NaN₃. The α-thrombin and α/β-thrombin liquid thrombin reagents were stored at 37°C for 0, 1, 2, 3, and 4 weeks and thrombin activities were determined using both a clotting assay and chromogenic assays, as described above.

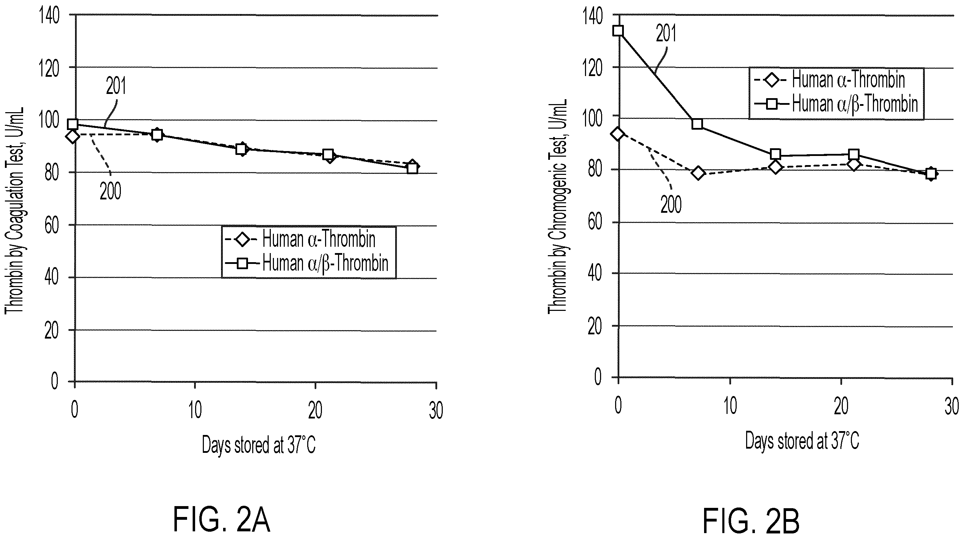

The results of the clotting assay for α-thrombin and α/β-thrombin liquid thrombin reagents are illustrated in Fig. 2A. The results of the chromogenic assay for α-thrombin and α/β-thrombin liquid thrombin reagents are illustrated in Fig. 2B.

As shown in Fig. 2A, as measured by the clotting assay, the α-thrombin liquid thrombin reagent 200 and the α/β-thrombin liquid thrombin reagent 201 that were stored for 0 weeks at 37°C had similar thrombin activities. Storage at 37°C for 1, 2, 3, or 4 weeks affected both α-thrombin and α/β-thrombin-containing liquid thrombin reagents equally as measured by the clotting assay.

As shown in Fig. 2B, as measured by the chromogenic assay, when stored for 0 weeks at 37°C, the α/β-thrombin liquid thrombin reagent 201 had 33% higher thrombin activity than the α-thrombin liquid thrombin reagent 200. The decline in activity for α/β-thrombin reagent was larger than that for α-thrombin liquid reagent after storage at 37°C for 1 to 2 weeks but was comparable to storage of the liquid thrombin reagent at 37°C for 2 to 4 weeks.

The test results shown in Figs. 2A and 2B indicate that the liquid thrombin reagent's thermal stability in carboxylic acid salts is moderated and under these conditions, β-thrombin is more labile (susceptible to cleavage) and has limited effect on α-thrombin activity.

### EFFECT OF FACTOR XA INHIBITOR APIXABAN ON LIQUID THROMBIN STABILITY

Factor Xa inhibitors inhibit or prevent thrombin autolysis. To show that Factor Xa inhibitors increase thrombin stability when included in the liquid thrombin reagents described herein, the following experiment was performed.

In this experiment, the effects of the direct factor Xa inhibitor apixaban was analyzed using a clotting assay. Liquid thrombin reagent containing low purity grade bovine thrombin was diluted to a final concentration of approximately 100 U/mL thrombin in an aqueous buffer containing 20 mM MES, pH 6.1, 100 mM NaCl, 25 mM sodium acetate, 25 mM sodium lactate, 5 mM CaCl₂, 0.5% BSA, and 0.4g/L NaN₃. Apixaban was dissolved in dimethyl sulfoxide (DMSO) and diluted to a final concentration of 500 ng/mL apixaban in a sample of the liquid thrombin reagent.

Liquid thrombin reagents with and without apixaban were stored at 45°C for 0, 2, 4, 7, 10 and 14 days, and tested in quadruplicate by the clotting assay after mixture with normal pooled plasma. The average clotting time for mixtures at each time point was compared to a baseline of the mixture stored at 45°C for 0 days.

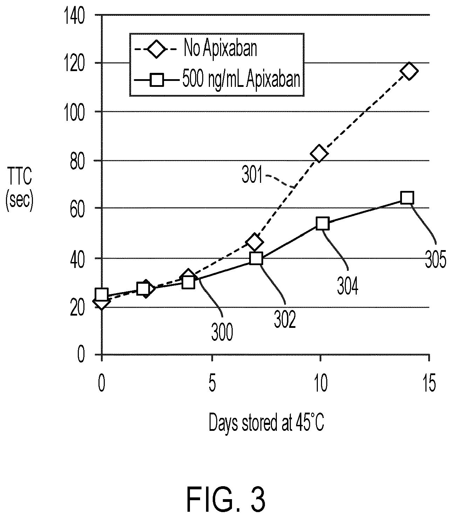

As shown in Fig. 3, the liquid reagent 301 that does not contain apixaban takes longer to clot the test sample (since the TTC is increased) than the liquid reagent that contains apixaban. This indicates that the apixaban contributes to the stability of the liquid thrombin reagent, since thrombin activity in the liquid reagent is not as impacted over time. Compared to 301, the loss in thrombin activity is pronounced after storage of the liquid thrombin reagent at 45°C for 7 (302), 10 (304), and 14 (305) days, demonstrating that apixaban improves the stability of the liquid thrombin reagent in the long term.

### EFFECT OF ALKYLATED GUANIDINES ON FACTOR XA ACTIVITY

To show that guanidine and derivatives thereof inhibit factor Xa activity and increase thrombin activity of the liquid thrombin reagents described herein, the following experiment was performed.

In this experiment, the effects of guanidine sulfate and the guanidine derivatives: 1-methylguanidine sulfate, 1,1-dimethylguanidine sulfate, 1,1-diethylguanidine, and N-benzyl, N-methylguanidine sulfate were analyzed using a chromogenic assay. A series of dilutions was prepared in water with guanidine or a guanidine derivative of the type described herein. 40 µL of each of the guanidine or guanidine derivatives were mixed for 20 to 60 seconds with 40 µL of 6 nanokatal per milliliter (nkat/mL) factor Xa diluted in 20mM HEPES, pH 7.5 buffer. After an incubation period of 150 to 180 seconds, 100 µL of the factor Xa chromogenic substrate S-2732 (1.5 mM) was added to each of the guanidine and factor Xa mixtures and was chromogenically measured at 405 nm milli-absorbance per minute (mAbs/min) after a 20 second delay.

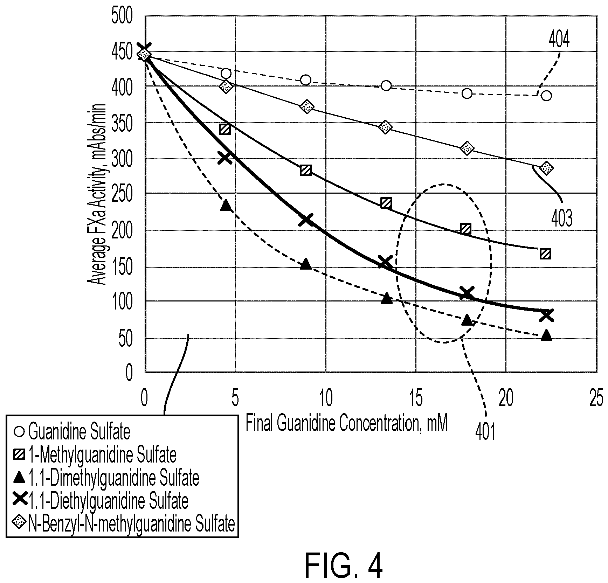

Mixtures containing the guanidine derivatives, such as alkylated guanidine derivatives, inhibited factor Xa activity. As shown in Fig. 4, the largest extent of factor Xa inhibition was seen in mixtures containing dialkylated guanidines 401 (dimethylguanidine sulfate and diethylguanidine sulfate). Mixtures containing a guanidine derivative containing a benzyl group 403 (N-benzyl N-methylguanidine sulfate) led to a reduced factor Xa inhibition as compared to the factor Xa inhibition in mixtures containing dialkylated guanidine derivatives 401. Mixtures containing guanidine sulfate 404 alone led to the least reduction in factor Xa inhibition. These results indicate that alkylated guanidines inhibit factor Xa activity. Direct factor Xa inhibitors, such as apixaban, support thrombin stability in liquid thrombin reagents by inhibiting factor Xa activity; and this experiment shows that a guanidine derivative will provide a similar stabilization effect on thrombin in a liquid thrombin reagent.

### EFFECT OF ALKYLATED GUANIDINE DERIVATIVES ON LIQUID THROMBIN REAGENT STABILITY

To show that alkylated guanidine derivatives stabilize thrombin activity of the liquid thrombin reagents described herein, the following experiment was performed.

The effects of guanidine derivatives were analyzed using a clotting assay. In this experiment, liquid thrombin reagents containing bovine thrombin were diluted to a final concentration of approximately 75 U/mL thrombin with an aqueous buffer comprising a MES buffer containing 20 mM MES, pH 6.3, and 0.1% ProCiin^{™} 300, with and without 25 mM of 1-methylguanidine (MMG), 25 mM of 1,1-dimethylguanidine (DMG) and 25 mM of 1,1-diethylguanidine (DEG).

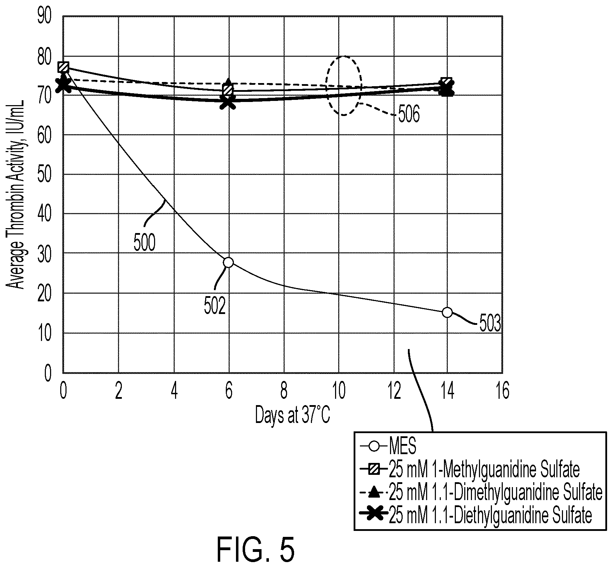

Liquid thrombin reagents of the type described herein containing guanidine derivatives, and one without a guanidine derivative, were stored at 37°C for 0, 7, and 14 days and tested using the clotting assay after mixing with normal pooled plasma. As shown in Fig. 5, liquid thrombin reagent without guanidine derivatives 500 (liquid thrombin plus MES buffer alone) stored at 37°C for 7 (502) or 14 (503) days resulted in a significant decrease of thrombin activity in the reagent. However, liquid thrombin reagents with any of the guanidine derivatives 506 (1,1 methylguanidine sulfate, 1,1 dimethylguanidine sulfate, or 1,1 diethylguanidine sulfate) stored at 37°C for 7 or 14 days resulted in little or no decrease in thrombin activity. This relatively consistent level of thrombin activity for liquid thrombin reagents containing the guanidine derivatives 506 demonstrates that the guanidine derivatives stabilize the liquid thrombin reagent.

### ALKYLATED GUANIDINE CONCENTRATIONS FOR THROMBIN STABILITY

To show examples of concentration of a guanidine derivative, such as alkylated guanidine, that stabilize thrombin activity in a liquid thrombin reagent, the following experiment was performed.

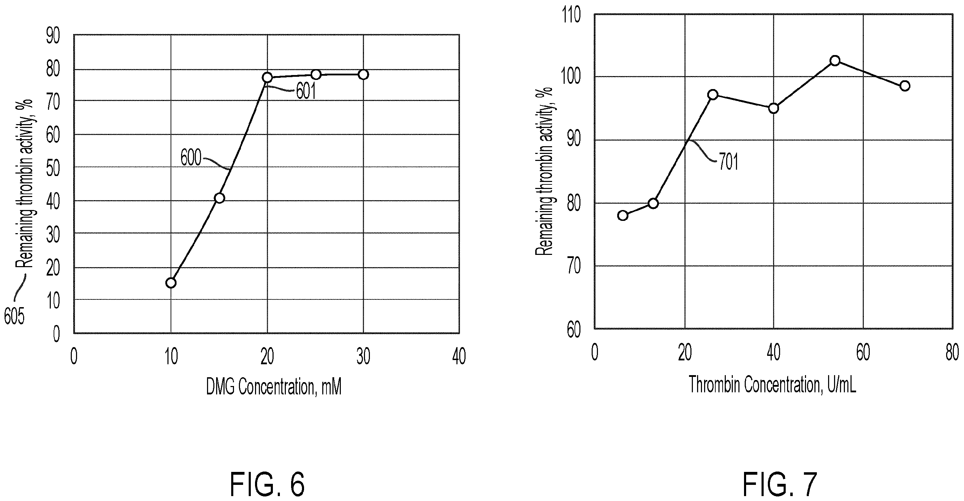

A series of liquid thrombin reagents containing dimethylguanidine (DMG) were analyzed using a clotting assay. A series of liquid thrombin reagents containing 7 U/mL liquid thrombin were prepared in an aqueous buffer containing 20 mM MES, pH 6.3, 0.1% ProCiin^{™} 300, and a final concentration of 5, 10, 15, 20, 25, or 30 mM DMG. The resulting liquid thrombin reagents were stored at 37°C for 20 days, and the remaining thrombin activity was compared to the liquid thrombin reagent containing the same concentration of guanidine derivative stored at 2 to 8°C for 20 days. As shown in Fig. 6, thrombin activity 600 in the liquid thrombin reagents that contained less than 20 mM DMG stored at 37°C for 20 days 601 declined (that is, the remaining amount of thrombin activity 605 decreased), whereas liquid thrombin reagents that contained 20 mM or more DMG had similar percentages of remaining thrombin activity. This retention of thrombin activity in liquid thrombin reagents containing DMG demonstrates that 20 mM DMG or more in a liquid thrombin reagent are examples of amounts of DMG to achieve thrombin stability for the temperature and time ranges considered.

### THROMBIN CONCENTRATIONS FOR LIQUID THROMBIN REAGENT STABILITY

To show an example amount of a guanidine derivative (in this example, DMG) to stabilize liquid thrombin, the following experiment was performed.

The effect of guanidine derivatives on a series of thrombin concentrations were analyzed using a clotting assay. A series of liquid thrombin reagents containing 30 mM DMG were prepared by diluting a 100 U/mL thrombin liquid thrombin reagent in MES buffer (containing 30 mM DMG, 20 mM MES, pH 6.3, and 0.1% ProCiin^{™} 300) with MES buffer to final concentrations of 70, 56, 42, 28, 14 or 7 U/mL thrombin. The liquid thrombin reagents were stored at 37°C for 7 days and tested for thrombin activity using the clotting assay. As shown in Fig. 7, activity 701 in the liquid thrombin reagents containing 30 mM DMG and less than about 20 U/mL liquid thrombin stored at 37°C for 7 days declined (that is, the remaining thrombin activity decreased), while liquid thrombin reagents that contained 30 mM DMG and more than about 20 U/mL thrombin did not decline significantly. This data demonstrates that a liquid thrombin reagent containing 20 U/mL thrombin or more and an alkylated guanidine is stable.

The assays that use the liquid thrombin reagents described herein can be performed manually or automatically. For example, the reagents may be manually or automatically activated, e.g., using robotics manipulators or flow-through fluidic devices to mix and test samples. Software applications may be used to analyze the test results to provide or calculate parameters, e.g., fibrinogen concentration or thrombin inhibitor concentration. The software applications may be used to create additional information that may be used, for example, to inform clinical actions. The use of the liquid thrombin reagents is not limited by the examples described in this disclosure, and the applicable assays are not limited by any particular system for implementation. The liquid thrombin reagents may be packaged and stored in bulk volumes or test volumes. The liquid thrombin reagents may be packaged and stored in any form, including forms for kits or cartridges. Below, several non-limiting example systems are described to illustrate example uses of the reagents in these systems.

In examples, the liquid thrombin reagent may be used as a Clauss fibrinogen assay reagent, a thrombin time assay reagent, or a diluted thrombin reagent for direct thrombin inhibitors. The Clauss fibrinogen assay is a quantitative, clot-based functional assay. The assay measures a rate of fibrinogen to fibrin conversion in a test sample, such as plasma or whole blood, under the influence of a thrombin reagent. Since, under these conditions, the fibrinogen content is rate limiting, the TTC can be used as a measure of fibrinogen concentration in the test sample. Specifically, the TTC is inversely proportional to the concentration of fibrinogen in the test sample. The thrombin time assay is a test that measures the time it takes for a fibrin clot to form in plasma of a blood sample. This time corresponds to the activity of fibrinogen.

The diagnostic test instruments / clinical analyzers and assays described herein may be controlled using computing systems or any other appropriate computing device having one or more processing devices, such as one or more microprocessors, one or more microcontrollers, or programmable logic such as one or more field-programmable gate arrays (FGPAs) or one or more application-specific integrated circuits (ASICs). The diagnostic test instruments / clinical analyzers described herein may execute one or more computer program products, e.g., one or more computer programs tangibly embodied in one or more information carriers, such as one or more non-transitory machine-readable media, to control the coagulation assays and to implement all or part of the processes described herein.

Elements of different implementations described herein may be combined to form other implementations not specifically set forth above. Elements may be left out of the structures described herein without adversely affecting their operation. Furthermore, various separate elements may be combined into one or more individual elements to perform the functions described herein.

## Claims

1. A composition that is liquid, the composition comprising:
thrombin; and
a constituent comprising one or more of guanidine derivatives or one or more anticoagulants.

2. The composition of claim 1, wherein the one or more guanidine derivatives comprise a mono-alkylated guanidine derivative.

3. The composition of claim 1, wherein the one or more guanidine derivatives comprise a dialkylated guanidine derivative.

4. The composition of claim 1, wherein the one or more guanidine derivatives comprise 1-methylguanidine, 1,1-dimethylguanidine, 1,1-diethylguanidine, or N-benzyl-N-methylguanidine, where N indicates a substituent on nitrogen.

5. The composition of claim 1, wherein the one or more guanidine derivatives comprise alkylated guanidine having a chemical structure represented by formula: 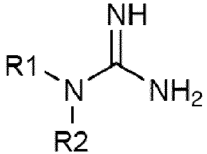
wherein R1 is H, methyl, ethyl, or benzyl;
wherein R2 is H, methyl, ethyl, or benzyl; and
wherein N is nitrogen and H is hydrogen.

6. The composition of claim 1, wherein the one or more guanidine derivatives comprise:
a mono-alkylated guanidine comprising an alkyl group having a length that is different from a length of an alkyl group of 1-methylguanidine; or
a dialkylated guanidine comprising an alkyl group having a length that is different from a length of an alkyl group of 1,1-dimethylguanidine or 1,1-diethylguanidine.

7. The composition of claim 1, wherein the one or more guanidine derivatives comprise a sulfate salt.

8. The composition of claim 1, wherein the one or more guanidine derivatives have a concentration in the composition of about 0.5 mM (millimolar) or greater.

9. The composition of claim 1, wherein the composition comprises a water-based solution.

10. The composition of claim 1, wherein the thrombin has a concentration in the composition of about 2 U/mL (units-per-milliliter) or greater or a concentration of about 20 U/mL (units-per-milliliter) or greater.

11. The composition of claim 1, wherein the one or more anticoagulants comprise rivaroxaban, apixaban, edoxaban, betrixaban, or a factor Xa anti-coagulant.

12. The composition of claim 1, further comprising:
a buffer that is aqueous.

13. The composition of claim 12, wherein the buffer comprises:
2-morpholin-4-ylethanesulfonic acid (MES); and
a preservative.

14. The composition of claim 12, wherein the buffer comprises:
one or more carboxylic acid derivatives.

15. The composition of claim 1, the composition being a liquid reagent for use as (i) a Clauss reagent, (ii) a thrombin time reagent, or (iii) a diluted thrombin reagent for direct thrombin inhibitors.

16. A method comprising:
forming a mixture comprising a liquid reagent and a sample, where the sample comprises whole blood, part of the whole blood, or a derivative of the whole blood, the liquid reagent comprising:
thrombin; and
a guanidine derivative; and
determining an amount of fibrinogen or an amount of anticoagulant directed against thrombin in the mixture.

17. The method of claim 16, wherein the liquid reagent and the mixture are in a cartridge; and
wherein the fibrinogen or thrombin inhibitor concentration is measured by an instrument in which the cartridge is inserted.

18. The method of claim 16, wherein the liquid reagent comprises a chromogenic substrate susceptible to cleavage by thrombin; and
wherein the thrombin reagent comprises a water-based solution.

19. A method of measuring thrombin inhibitor in a sample, the method comprising:
forming a mixture comprising a liquid reagent and the sample, where the sample comprises a thrombin inhibitor in whole blood, part of the whole blood, or a derivative of the whole blood, the liquid reagent comprising:
thrombin; and
a guanidine derivative; and
determining an amount of thrombin inhibitor in the mixture.

20. The method of claim 19, wherein the guanidine derivative comprises 1-methylguanidine, 1,1-dimethylguanidine, 1,1-diethylguanidine, or N-benzyl-N-methylguanidine.
